# EUROPEAN PATENT APPLICATION

(11) **EP 4 306 087 A1**
(43) Date of publication of application: **17.01.2024**
(21) Application number: 22766839.9
(22) Date of filing: 24.02.2022
(51) Int. Cl.: A61F 7/08, A61F 7/10

(54) **HOLDER FOR HEATING AND COOLING TOOL**

(30) Priority: 08.03.2021 JP 2021036139
(71) Applicant: Kobayashi Pharmaceutical Co., Ltd., Chuo-ku Osaka-shi, Osaka 541-0045 (JP)
(72) Inventor: INOUE, Nobuto, Ibaraki-shi, Osaka 567-0057 (JP)
(74) Representative: Hasegawa, Kan
(86) International application number: PCT/JP2022/007618
(87) International publication number: WO 2022/190880

(57) **Abstract**

To provide a holder which is for a heating and cooling tool and which fits on the upper arm and can provide a stable heating or cooling effect to skin.

This holder for a heating and cooling tool is mounted on the upper arm, and comprises: a holding part having a pocket in which a heater or cooler is accommodated; and a belt joined to the holding part so that an insertion space into which the upper arm is inserted is formed between the holding part and the belt, wherein the holding part is made of a material that does not substantially expand or contract, and the belt is made of an elastic material to allow the belt to conform to changes in the thickness of the upper arm accompanying bending and straightening of the elbow.

## Description

### TECHNICAL FIELD

The present invention relates to a holder for a heating and cooling tool mounted on an upper arm.

### BACKGROUND ART

There is known a holder for a heating and cooling tool that has a pocket and is attached to a part of a body of a user in a state where a heating tool or a cooling tool is accommodated in the pocket. Patent Document 1 discloses an attachment for a heating and cooling tool to be attached to a wrist of a user as an example of a holder for a heating and cooling tool. The attachment for a heating and cooling tool is made of a material that does not substantially stretch and contract as a whole.

### PRIOR ART DOCUMENT

### PATENT DOCUMENT

Patent Document 1: Japanese Patent Laid-open Publication No. 2018-389

### SUMMARY OF THE INVENTION

### PROBLEMS TO BE SOLVED BY THE INVENTION

When the attachment for a heating and cooling tool is mounted on the upper arm, since the thickness of the upper arm greatly changes as the elbow is bent and stretched, the attachment for a heating and cooling tool is preferably made of a stretchable material. On the other hand, when the attachment for a heating and cooling tool is made of a stretchable material, the thickness of the fabric of the pocket and the portion in contact with the skin of the user changes with the change in the thickness of the upper arm, so that the heating effect or the cooling effect applied to the skin is not stabilized.

An object of the present invention is to provide a holder for a heating and cooling tool that fits an upper arm and can impart a stable heating effect or cooling effect to a skin.

### MEANS FOR SOLVING THE PROBLEM

A holder for a heating and cooling tool according to a first aspect of the present invention is a holder for a heating and cooling tool which is mounted on an upper arm, the holder including: a holding portion having a pocket in which a heating tool or cooling tool is accommodated; and a belt joined to the holding portion so that an insertion space into which the upper arm is inserted is formed between the holding portion and the belt, in which the holding portion is made of a material that does not substantially stretch and contract, and in which the belt is made of a material having stretchability to follow a change in a thickness of the upper arm accompanying bending and stretching of an elbow.

The thickness of the upper arm greatly changes as the elbow is bent and stretched. Specifically, the upper arm is thicker when the elbow is bent than when the elbow is extended. According to the holder for a heating and cooling tool, the belt stretches and contracts so as to fit the upper arm even when the thickness of the upper arm changes. On the other hand, since the holding portion is made of a material that does not substantially stretch and contract, the thickness of the holding portion does not substantially change even when the thickness of the upper arm changes. Therefore, even when the thickness of the upper arm changes, the heating effect or the cooling effect applied to the skin is stabilized. As described above, according to the holder for a heating and cooling tool, even when the thickness of the upper arm changes, it is possible to fit the upper arm and to impart a stable heating effect or cooling effect to the skin.

A holder for a heating and cooling tool according to a second aspect of the present invention is the holder for a heating and cooling tool according to the first aspect, further including an adjustment portion that protrudes from the belt and is joined to the belt to adjust a length of the belt.

According to the holder for a heating and cooling tool, since the adjustment portion protrudes from the belt, the entire belt in the longitudinal direction can be selected as the adjustment range of the length of the belt. Therefore, it can be worn on upper arms of various sizes.

A holder for a heating and cooling tool according to a third aspect of the present invention is the holder for a heating and cooling tool according to the second aspect, in which the belt includes one of a loop and a hook of a hook-and-loop fastener, and in which the adjustment portion includes the other of the loop and the hook of the hook-and-loop fastener.

According to the holder for a heating and cooling tool, the belt and the adjustment portion are firmly joined. Therefore, the holder for a heating and cooling tool is hardly detached from the upper arm.

### ADVANTAGES OF THE INVENTION

According to the holder for a heating and cooling tool of the present invention, it is possible to fit the upper arm and to impart a stable heating effect or cooling effect to the skin.

### BRIEF DESCRIPTION OF THE DRAWINGS

Fig. 1 is a front view of a holder for a heating and cooling tool according to an embodiment.
Fig. 2 is a rear view of the holder for a heating and cooling tool of Fig. 1.
Fig. 3 is a side view of the holder for a heating and cooling tool of Fig. 1.
Fig. 4 is a perspective view of a state in which the holder for a heating and cooling tool of Fig. 1 is attached to an upper arm.
Fig. 5 is an enlarged view of a surface of a stretchable fabric in an unstretched state.
Fig. 6 is an enlarged view of a surface of a stretchable fabric in a state of being stretched by 180%.

### EMBODIMENTS OF THE INVENTION

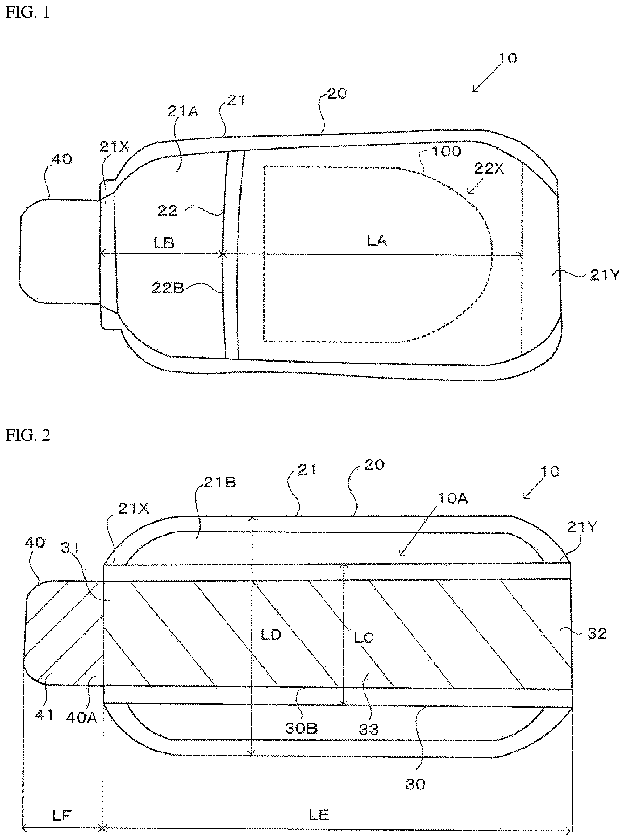

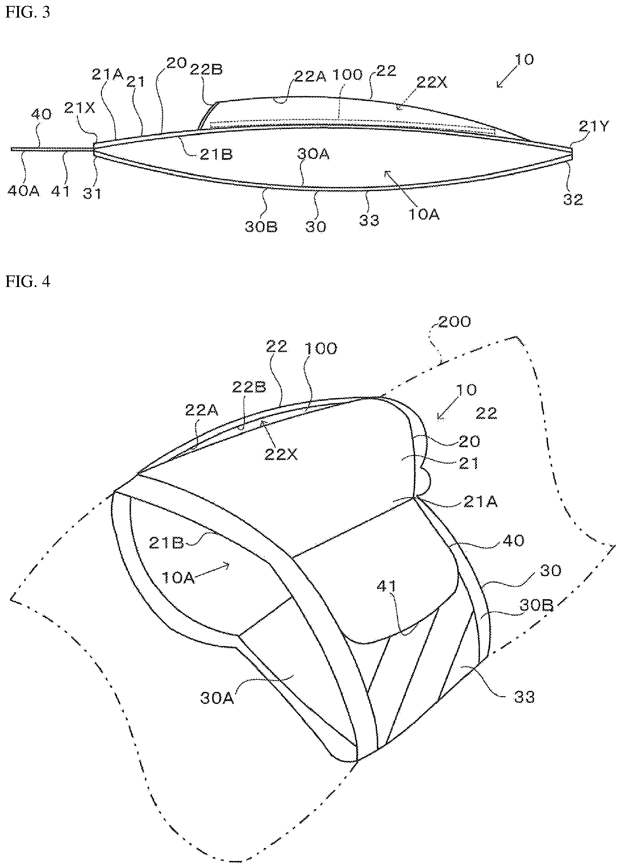

Hereinafter, an embodiment of a holder for a heating and cooling tool according to the present invention will be described with reference to the drawings. FIG. 1 is a front view of a holder 10 for a heating and cooling tool according to the embodiment. FIG. 2 is a rear view of the holder 10 for a heating and cooling tool. FIG. 3 is a side view of the holder 10 for a heating and cooling tool. Hereinafter, in a front view of the holder 10 for a heating and cooling tool, a longitudinal direction of the holder 10 for a heating and cooling tool is referred to as a left-right direction, and in a front view, a direction orthogonal to the left-right direction is referred to as an up-down direction.

### <1. Overall Configuration of Holder for Heating and Cooling Tool>

The holder 10 for a heating and cooling tool illustrated in FIGS. 1, 2, and 3 is attached to an upper arm 200 (see FIG. 4) of a user in a state where a heating tool or a cooling tool is accommodated. The heating tool is, for example, a warmer. The warmer is, for example, a chemical warmer, an oil warmer, or a rechargeable warmer. The cooling tool is, for example, a refrigerant or a coolant. The holder 10 for a heating and cooling tool of the present embodiment accommodates, as a heating tool, a sheet-like chemical warmer 100 (hereinafter referred to as a "warmer 100") that generates heat by reacting with oxygen in the air. Main elements constituting the holder 10 for a heating and cooling tool are a holding portion 20, a belt 30, and an adjustment portion 40.

### <1-1. Holding Portion>

The holding portion 20 has a base portion 21 and a pocket 22 attached to the base portion 21. The material constituting the holding portion 20 is made of a material that does not substantially stretch and contract even when the thickness of the upper arm 200 changes in the state of being worn on the upper arm 200 of the user. The material constituting the holding portion 20 is, for example, polyester, nylon, or cotton. In the present embodiment, "being not substantially stretching and contracting" means that, for example, when the fabric is stretched by 1 cm in a predetermined direction, a length in the predetermined direction of a portion that breaks or returns to a state before stretching in the stretched portion is less than 95% (0.95 cm).

The shape of the base portion 21 can be arbitrarily selected. In the present embodiment, the base portion 21 has a shape in which portions corresponding to four corners of a rectangle are chamfered in a front view. The pocket 22 for accommodating the warmer 100 is attached to a surface 21A of the base portion 21. The pocket 22 is formed by attaching a fabric different from the fabric constituting the base portion 21 to the surface 21A of the base portion 21. From the viewpoint of preventing the warmer 100 from falling off, the pocket 22 is sewn to the base portion 21 except for the portion where an opening 22B is formed. An accommodation space 22X for accommodating the warmer 100 is formed between an inner surface 22A (see FIG. 3) of the pocket 22 and the surface 21A of the base portion 21. The accommodation space 22X communicates with the external space via the opening 22B. The warmer 100 is taken in and out of the accommodation space 22X through the opening 22B.

A ratio RA of a maximum length LB between the opening 22B of the pocket 22 and one end portion 21X of the base portion 21 in the left-right direction to a maximum length LA in the left-right direction of the pocket 22 can be arbitrarily selected. When the length LA is longer than the length LB, the pocket 22 can be formed larger, so that the warmer 100 having various sizes can be accommodated in the pocket 22. In a case where the length LA is longer than the length LB, since the pocket 22 exists over a wide range in the left-right direction, when the holder 10 for a heating and cooling tool is attached to the upper arm 200, the inside of the pocket 22 and the warmer 100 accommodated in the pocket 22 are curved along the upper arm 200. Since the warmer 100 fits the upper arm 200, the state in which the warmer 100 is accommodated in the pocket 22 is easily maintained. From such a viewpoint, the ratio RA is preferably less than 100%, and more preferably less than 50%. In the present embodiment, the length LA is 11 cm, and the length LB is 5 cm. That is, the ratio RA is 45.5%.

### <1-2. Belt>

The belt 30 illustrated in FIG. 2 has, for example, a rectangular shape, and is joined to the base portion 21 such that an insertion space 10A into which the upper arm 200 is inserted is formed between a front surface 30A (see FIG. 3) and the back surface 21B of the base portion 21. One end portion 31 of the belt 30 in the left-right direction is joined to one end portion 21X of the base portion 21 in the left-right direction. The other end portion 32 of the belt 30 in the left-right direction is joined to the other end portion 21Y of the base portion 21 in the left-right direction. The thickness of the upper arm 200 greatly changes as the elbow is bent and stretched. Specifically, the upper arm 200 is thicker when the elbow is bent than when the elbow is extended. Therefore, the belt 30 is made of a stretchable fabric containing a material having stretchability so as to follow a change in the thickness of the upper arm 200 accompanying bending and stretching of the elbow of the user in a state of being worn on the upper arm 200 of the user. When the user bends the elbow, the belt 30 stretches more than when the user stretches the elbow. The belt 30 extends as the angle between the forearm and the upper arm 200 is smaller. When the user stretches the elbow, the belt 30 contracts more than when the user bends the elbow. The belt 30 contracts as the angle between the forearm and the upper arm 200 of the user increases. In the present embodiment, having stretchability means that, for example, when a fabric is stretched by 1 cm in a predetermined direction, a length in the predetermined direction of a portion that returns to a state before stretching in the stretched portion is 95% (0.95 cm) or more. Even when the stretchable fabric contains a material that does not substantially stretch and contract, the stretchable fabric is configured to have stretchability as a whole. In a first example, the stretchable fabric is made of a single material having stretchability. The material having stretchability is, for example, polyurethane, natural rubber, or chloroprene rubber. In a second example, the stretchable fabric is made of a plurality of materials having stretchability, or is made of a material having stretchability and a material that does not substantially stretch and contract. The material that does not substantially stretch and contract is, for example, nylon. In a third example, the belt 30 is configured by bonding a stretchable fabric and a non-stretchable fabric that does not substantially stretch and contract. The stretchable fabric is the stretchable fabric shown in the first example or the second example. The material constituting the non-stretchable fabric is, for example, polyester. Even in the case of the third example, the belt 30 is configured to have stretchability as a whole. According to the second example and the third example, the strength of the belt 30 is increased. Further, according to the second example and the third example, the stretchability of the belt 30 can be arbitrarily adjusted. Note that the belt 30 only needs to have stretchability at least in a direction along the left-right direction, and may have stretchability in a direction intersecting the left-right direction in a front view.

The relationship between a maximum length LC of the belt 30 in the up-down direction and a maximum length LD of the base portion 21 in the up-down direction can be arbitrarily selected. Since the belt 30 is made of a stretchable material, the length LC is preferably shorter than the length LD. When the length LC is shorter than the length LD, the belt 30 easily stretches and contracts, and thus, options of materials constituting the belt 30 are widened. In one example, the length LC is 5 cm and the length LD is 9 cm.

A loop 33 of a hook-and-loop fastener is attached to a back surface 30B of the belt 30. A hatched portion of the belt 30 in FIG. 2 indicates an area where the loop 33 exists. In the present embodiment, the loop 33 is arranged over substantially the entire belt 30 in the left-right direction.

### <1-3. Adjustment Portion>

The adjustment portion 40 protrudes from the end portion 31 of the belt 30 and the end portion 21X of the base portion 21, and is joined to the belt 30 to adjust the length of the belt 30 wound around the upper arm 200. The adjustment portion 40 has, for example, a rectangular shape in which a portion corresponding to a corner located on the opposite side of the belt 30 is chamfered. A hook 41 of a hook-and-loop fastener to be joined to the loop 33 is attached to a back surface 40A of the adjustment portion 40. In FIG. 2, a hatched portion of the adjustment portion 40 indicates a region where the hook 41 exists. The hook 41 is arranged over substantially the entire back surface 40A of the adjustment portion 40.

A ratio RB of a maximum length LF of the adjustment portion 40 in the left-right direction to a maximum length LE of the belt 30 in the left-right direction can be arbitrarily selected. Preferably, the ratio RB is 17% or more so that the user can easily hold the adjustment portion 40 in a state where the upper arm 200 is inserted into the insertion space 10A. In the present embodiment, the length LE is 17.5 cm, and the length LF is 3 cm. That is, the ratio RB is 17.1%.

### <2. Method of Using Holder for Heating and Cooling Tool>

An example of a method of using the holder 10 for a heating and cooling tool will be described with reference to FIGS. 3 and 4.

As shown in Fig. 3, the warmer 100 is stored in the accommodation space 22X through the opening 22B. The user inserts the hand, the forearm, the elbow, and the upper arm 200 into the insertion space 10A in this order. When the holder 10 for a heating and cooling tool reaches a desired position of the upper arm 200, the user pulls the hook 41 of the adjustment portion 40 and joins the hook 41 to the loop 33 of the belt 30 so that the holder 10 for a heating and cooling tool is not displaced with respect to the upper arm 200. A portion of the belt 30 closer to the end portion 31 than a portion joined to the adjustment portion 40 is positioned between the upper arm 200 and the back surface 21B of the base portion 21 in a bent state. When adjustment of the length of the belt 30 by the adjustment portion 40 is not necessary, the hook 41 and the loop 33 are not joined. When the holder 10 for a heating and cooling tool is attached to the upper arm, a heating effect is applied to the upper arm 200 via the back surface 21B of the base portion 21.

### <3. Functions and Effects of Present Embodiment>

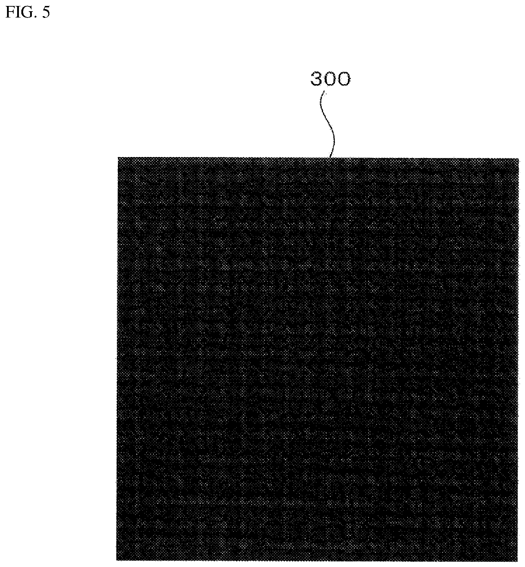

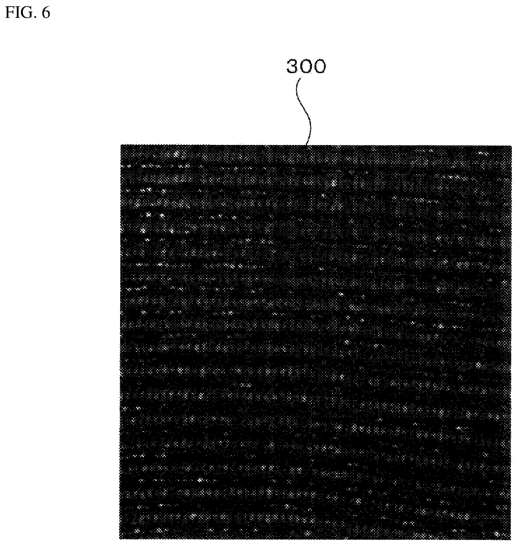

Fig. 5 is an enlarged view of a surface of a stretchable fabric 300 in an unstretched state. Fig. 6 is an enlarged view of a surface of the stretchable fabric 300 in a state of being stretched by 180%. The stretchable fabric 300 has a rougher basis weight and a thinner thickness in a stretched state than in an unstretched state. Therefore, for example, when the stretchable fabric 300 is used as the material constituting the holding portion 20, the basis weight and the thickness of the portion constituting the base portion 21 in the stretchable fabric 300 change with the change in the thickness of the upper arm 200, so that the heating effect or the cooling effect applied to the skin of the user from the heating tool or the cooling tool accommodated in the pocket 22 is not stabilized. Furthermore, the air permeability of the pocket 22 also changes as the basis weight and the thickness of the portion constituting the pocket 22 in the stretchable fabric 300 change. When the warmer 100 is a chemical warmer that generates heat by reacting with oxygen in the air, the heat generation state is not stabilized and the temperature of the warmer 100 is not stabilized due to a change in the air permeability of the pocket 22.

According to the holder 10 for a heating and cooling tool, since the belt 30 is formed of the stretchable fabric 300, the belt 30 stretches and contracts so as to fit the upper arm 200 even when the thickness of the upper arm 200 changes. On the other hand, since the holding portion 20 is made of a material that does not substantially stretch and contract, the thickness does not substantially change even when the thickness of the upper arm 200 changes. Therefore, even when the thickness of the upper arm 200 changes, the heating effect or the cooling effect applied to the skin is stabilized. As described above, according to the holder 10 for a heating and cooling tool, even when the thickness of the upper arm 200 changes, it is possible to fit the upper arm 200 and stably apply the heating effect or the cooling effect to the skin of the user.

### <4. Effects of Present Embodiment>

With the holder 10 for a heating and cooling tool configured as described above, the following effects can be further obtained.

<4-1> According to the holder 10 for a heating and cooling tool, since the adjustment portion 40 protrudes from the belt 30, the entire belt 30 in the longitudinal direction can be selected as the adjustment range of the length of the belt 30. Therefore, it can be mounted on the upper arm 200 of various sizes.

<4-2> In the holder 10 for a heating and cooling tool, the hook 41 of the adjustment portion 40 and the loop 33 of the belt 30 are joined in a state of being attached to the upper arm 200. Since the belt 30 and the adjustment portion 40 are firmly joined, the holder 10 for a heating and cooling tool is hardly detached from the upper arm 200.

<4-3> Since the volume of the accommodation space 22X does not substantially change even when the thickness of the upper arm 200 changes, the amount of oxygen existing in the accommodation space 22X is constant. When the warmer 100 is a chemical warmer that generates heat by reacting with oxygen in the air as in the present embodiment, the heat generation state of the warmer 100 is stabilized, so that the temperature of the warmer 100 is stabilized.

<4-4> Since the holding portion 20 is made of a material that does not substantially stretch and contract, even if the thickness of the upper arm 200 changes, the air permeability of the accommodation space 22X through the base portion 21 and the pocket 22 does not substantially change. When the warmer 100 is a chemical warmer, the heating state of the warmer 100 is stabilized, so that the temperature of the warmer 100 is stabilized.

### <5. Modifications>

The above embodiment is an example of a form that can be taken by the holder for a heating and cooling tool according to the present invention, and is not intended to limit the form. The holder for a heating and cooling tool according to the present invention may take a form different from the form exemplified in the embodiment. An example thereof is a form in which a part of the configuration of the embodiment is replaced, changed, or omitted, or a form in which a new configuration is added to the embodiment. Some examples of modifications of the embodiment will be described below.

<5-1> In the above embodiment, the loop 33 is arranged on the belt 30 and the hook 41 is arranged on the adjustment portion 40, but the hook 41 may be arranged on the belt 30 and the loop 33 may be arranged on the adjustment portion 40.

<5-2> In the above embodiment, the adjustment portion 40 protrudes from the belt 30, but the adjustment portion 40 may be arranged on the back surface 30B of the belt 30.

### DESCRIPTION OF REFERENCE SIGNS

10: Holder for heating and cooling tool
10A: Insertion space
20: Holding portion
22: Pocket
30: Belt
33: Loop
40: Adjustment portion
41: Hook
100: Warmer (heating tool)
200: Upper arm

## Claims

1. A holder for a heating and cooling tool which is mounted on an upper arm, the holder comprising:
a holding portion having a pocket in which a heating tool or cooling tool is accommodated; and
a belt joined to the holding portion so that an insertion space into which the upper arm is inserted is formed between the holding portion and the belt,
wherein the holding portion is made of a material that does not substantially stretch and contract, and
wherein the belt is made of a material having stretchability to follow a change in a thickness of the upper arm accompanying bending and stretching of an elbow.

2. The holder for a heating and cooling tool according to claim 1, further comprising an adjustment portion that protrudes from the belt and is joined to the belt to adjust a length of the belt.

3. The holder for a heating and cooling tool according to claim 2,
wherein the belt includes one of a loop and a hook of a hook-and-loop fastener, and
wherein the adjustment portion includes another of the loop and the hook of the hook-and-loop fastener.
